# EUROPEAN PATENT APPLICATION

(11) **EP 4 556 076 A1**
(43) Date of publication of application: **21.05.2025**
(21) Application number: 24213175.3
(22) Date of filing: 15.11.2024
(51) Int. Cl.: A61Q 1/04, A61Q 1/06, A61K 8/60

(54) **FLUID COSMETIC LIP BALM AND PROCESS FOR MAKING SAID COSMETIC BALM**

(30) Priority: 17.11.2023 IT 202300024477
(71) Applicant: Regi S.r.l., 26010 Bagnolo Cremasco (IT)
(72) Inventor: SANTORO, Barbara, 26010 Casaletto Ceredano (IT)
(74) Representative: Lunati & Mazzoni S.r.L.

(57) **Abstract**

A fluid cosmetic balm (1) for lips is provided, composed of an anhydrous cosmetic formulation (1a) comprising at least Stevioside (2) capable of imparting at least a sweetening effect to the formulation (1), with the Stevioside (2) being finely dispersed in the formulation (1).

## Description

The present invention relates to a fluid cosmetic lip balm and a process for making said cosmetic balm of the type specified in the preamble of the independent claims. Currently, there are products designed to be used for application to the lips. These products are divided into two categories: lip sticks and fluid lip balms. In particular, these products can perform different functions depending on the intended use. Fluid lip balms have been developed that serve a protective function, to prevent the cracking of the skin of the lips, or to prevent diseases such as stomatitis.

Such products are, for example, described in patent applications: US-A-2012/070533; US-A-2022/354756 and CN-A-104274581.

Another category of fluid lip balms, on the other hand, performs an aesthetic function. In particular, this category of fluid lip balms mainly serves to apply a formulation containing a mixture of pigments that imparts a particular colouring to the lips. Typical examples of fluid lip balms belonging to this category are lipsticks. Other types of fluid lip balms may perform aesthetic functions of a different nature such as, for example, imparting a visual effect to the lips, such as a gloss effect, to make the lips shinier. Examples of this type of fluid lip balm are lip glosses. Another category of fluid lip balms includes aromatic substances capable of giving the product a fragrance and, in certain formulations, also a pleasant flavour.

Fluid lip balms have been developed that combine the aesthetic, protective, and additive functions with the addition of aromas capable of imparting a fragrance or aroma to the product.

In particular, both lipsticks and lip glosses, in addition to the aesthetic function, can perform protective functions and include aromas through the preparation of mixed formulations.

The known technique described has some important drawbacks.

In fact, the use and maintenance of such balms are not pleasant or comfortable for the user.

In this situation, the technical task underlying the present invention is to design a cosmetic formulation and a process for making said cosmetic formulation capable of substantially overcoming at least part of the cited drawbacks.

As part of said technical task, an important objective of the invention is to achieve a fluid cosmetic lip balm and a process for making said cosmetic balm that is pleasant and comfortable in application and use.

The technical task and specified objectives are achieved by a fluid cosmetic lip balm and a process for making said cosmetic balm as claimed in the accompanying independent claims.

Preferred technical solutions are highlighted in the dependent claims.

The characteristics and advantages of the invention are clarified below throughout the detailed description of preferred embodiments of the invention, with reference to the accompanying drawings, wherein:

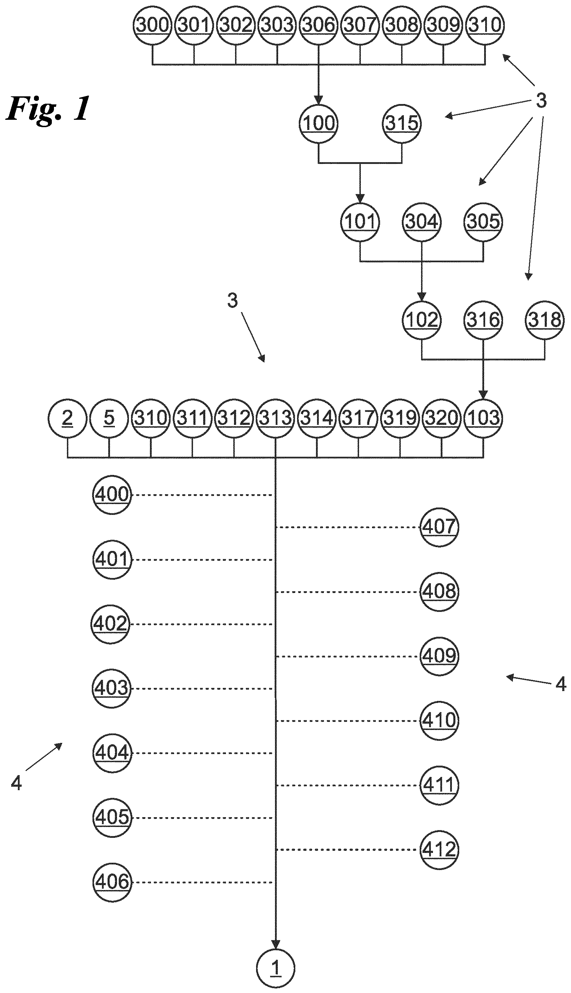

**Fig. 1** shows a diagram of the process for making a cosmetic balm according to the invention.

In this document, measurements, values, shapes, and geometric references (such as perpendicularity and parallelism), when associated with words such as "approximately" or other similar terms such as "virtually" or "substantially", are to be understood as subject to measurement errors or inaccuracies due to production and/or manufacturing errors and, above all, subject to slight deviations from the value, measurement, shape, or geometric reference with which they are associated. For example, such terms, if associated with a value, preferably indicate a deviation of no more than 10% from the value itself. Furthermore, when used, terms such as "first", "second", "upper", "lower", "primary", and "secondary" do not necessarily identify an order, a priority of relation, or relative position, but may simply be used to more clearly distinguish different components one from the other.

Unless otherwise specified, as results from the following description, terms such as "processing", "computing", "determination", "computation", or similar are considered to refer to the action and/or processes of a computer or similar electronic computation device that manipulates and/or transforms data represented as physical quantities, such as electronic magnitudes of records of a computing system and/or memories into other data similarly represented as physical quantities within computer systems, records, or other storage, transmission, or information display devices.

The measurements and data reported in this text are to be considered, unless otherwise indicated, as performed according to the indications of Good Manufacturing Practice GMP UNI EN ISO 22716:2013.

With reference to the Figures, the cosmetic balm according to the invention is globally denoted by the number 1. It is a cosmetic balm intended for use in a product for application to the lips. In particular, the product is preferably a fluid cosmetic lip balm.

It preferably consists, preferably entirely consists, of a formulation 1a.

Said formulation 1a preferably has an anhydrous base, i.e., free from water. The cosmetic balm 1 and the formulation 1a are therefore substantially preferably free from water, meaning they contain a water content lower than 3%, more preferably less than 1%, and even more preferably, water is not intentionally added to the components it is made of.

The formulation 1a preferably includes Palmitoyl Tripeptide-1 **5.** The addition of this substance to the formulation 1 is advantageous because it is capable of imparting at least a volumizing, moisturizing, and wrinkle-reducing effect to the formulation 1. The formulation 1a preferably comprises a plurality of components **3.** These components are able to impart different properties to the formulation 1 from a rheological, chemical, and cosmetic point of view. In particular, they are capable of imparting to the formulation 1a active, emollient, skin-conditioning, viscosity-regulating, surfactant, thickening, fragrant, makeup, emulsifying, rheological modifier, antioxidant, texturizing, plumping, moisturizing, and soothing effects. The components 3 preferably include one or more of the following elements: Hydrogenated Polyisobutene **300,** Diisostearyl Malate **301,** Butyrospermum Parkii Butter **302,** Polybutene **303,** Microcrystalline Wax **304,** Synthetic Wax **305,** Octyldodecanol **306,** Hydrogenated Poly(C6-14 Olefins) **307,** Polyglyceryl-2 Triisostearate **308,** Phytosteryl/lsostearyl/Cetyl/Stearyl/Behenyl Dimer Dilinoleate **309,** Tocopherol **310,** Tribehenin **311,** Ethylhexyl Palmitate **312,** Sorbitan Isostearate **313,** Lactic Acid **314,** Disteardimonium Hectorite **315,** Tocopheryl Acetate **316,** Orbignya Oleifera Seed Oil **317,** Synthetic Fluorphlogopite **318,** Tetrahexyldecyl Ascorbate **319,** Theobroma Grandiflorum Seed Butter **320.** Hydrogenated Polyisobutene 300 serves the function of imparting emollient, skin-conditioning, and viscosity-controlling properties to the formulation 1a. Diisostearyl Malate 301 also serves an emollient and skin-conditioning function, and it also acts as a surfactant.

Butyrospermum Parkii Butter 302, also known as Shea Butter, serves the function of an active ingredient. Indeed, it provides skin protection and hydration. Polybutene 303 serves a thickening function.

Microcrystalline Wax 304 and Synthetic Wax 305 serve as stabilizing and thickening agents.

Octyldodecanol 306 serves as an emollient and fragrance agent.

Hydrogenated Poly(C6-14 Olefins) 307 and Phytosteryl/lsostearyl/Cetyl/Stearyl/Behenyl Dimer Dilinoleate 309 serve the function of ester and lubricant.

Polyglyceryl-2 Triisostearate 308 serves the function of emulsifier.

Tocopherol 310, also known as Vitamin E, serves the function of antioxidant.

The mixture of Palmitoyl Tripeptide-1 5, Tribehenin 311, Ethylhexyl Palmitate 312, and Sorbitan Isostearate 313 serves the function of volumizing, moisturizing, and wrinkle-reducing.

Lactic Acid 314 serves the function of pH modifier, emollient, and humectant. Orbignya Oleifera Seed Oil 317 (known as Babassu oil) serves the function of emollient.

Theobroma Grandiflorum Seed Butter 320 (also known as Cupuaçu Butter) serves the function of skin conditioning agent.

Disteardimonium Hectorite 315 serves the function of rheological modifier. Tocopheryl Acetate 316 and Tetrahexyldecyl Ascorbate 319 serve the function of antioxidant and skin conditioner.

Finally, Synthetic Fluorphlogopite 318 serves the function of texturizing agent. The formulation 1a advantageously includes at least Stevioside **2.** It is designed to impart at least a sweetening effect to the formulation 1a.

The Stevioside 2 is also advantageously finely dispersed in the formulation 1a. The introduction of Stevioside 2 into the formulation 1a in a finely dispersed form is advantageous as it prevents the "scrub" effect when the formulation 1a is used in a fluid lip balm.

In this regard, the invention comprises a new process for making the cosmetic formulation 1a designed for use in a product for application to the lips. The process includes at least one phase of mixing a plurality of components 3.

Each mixing phase is preferably preceded by the weighing of the substances to be mixed.

In particular, the amounts added of each substance ensure that the corresponding weight percentages in the formulation 1a at the end of the process are within the ranges of percentage values reported below. Therefore, the weight percentage of Palmitoyl Tripeptide-1 5 in the formulation 1a is preferably less than 1%. The percentage of Hydrogenated Polyisobutene 300 is preferably between 30% and 40%, more preferably between 32% and 38%. The percentage of Diisostearyl Malate 301 is preferably between 20% and 30%, more preferably between 22% and 28%. The percentage of Butyrospermum Parkii Butter 302 is preferably between 9% and 15%, more preferably between 10% and 14%. The percentage of Polybutene 303 is preferably between 5% and 15%, more preferably between 7% and 14%. The percentage of Microcrystalline Wax 304 is preferably between 2% and 7%, more preferably between 3% and 6%. The percentage of Synthetic Wax 305 is preferably between 1% and 5% by weight, more preferably between 2% and 4%. The weight percentage of Octyldodecanol 306 is preferably between 1% and 4%, more preferably between 2% and 3%. The percentage of Hydrogenated Poly(C6-14 Olefins) 307 is preferably between 1% and 4%. The percentage of Polyglyceryl-2 Triisostearate 308 is preferably between 1% and 4%. The percentage of Phytosteryl/lsostearyl/Cetyl/Stearyl/Behenyl Dimer Dilinoleate 309 is preferably less than 2%, more preferably less than 1%. The percentage of Disteardimonium Hectorite 315 is preferably less than 2%, more preferably less than 1%. The percentage of Ethylhexyl Palmitate 312 is preferably less than 2%, more preferably less than 1%. The percentage of Tocopherol 310 is preferably less than 1%. The percentage of Tribehenin 311 is preferably less than 1%. The percentage of Sorbitan Isostearate 313 is preferably less than 1%. The percentage of Lactic Acid 314 is preferably less than 1%. The percentage of Tocopheryl Acetate 316 is preferably less than 1%. The percentage of Orbignya Oleifera Seed Oil 317 is preferably less than 1%.

The percentage of Synthetic Fluorphlogopite 318 is preferably less than 1%.

The percentage of Tetrahexyldecyl Ascorbate 319 is preferably less than 1%.

The percentage of Theobroma Grandiflorum Seed Butter 320 is preferably less than 1%.

Each phase is preferably conducted at a pressure between 700 mbar and 1100 mbar. More preferably, the pressure ranges between 800 mbar and 1000 mbar.

In particular, the process preferably includes a first mixing phase. In this phase, a first mixture **100** is prepared. It is obtained by mixing Diisostearyl Malate 301, Hydrogenated Polyisobutene 300, Polybutene 303, Butyrospermum Parkii Butter 302, Octyldodecanol 306, Hydrogenated Poly(C6-14 Olefins) 307, Polyglyceryl-2 Triisostearate 308, Phytosteryl/lsostearyl/Cetyl/Stearyl/Behenyl Dimer Dilinoleate 309, and Tocopherol 310.

This first mixing phase takes place at a temperature preferably between 75°C and 95°C, more preferably between 80°C and 90°C.

The mixing phases can be conducted inside a turboemulsifier. It consists of a mixing chamber provided with rotating blades for mixing and counter-blades. In this first mixing phase, the blades can be rotated at a speed sufficient to allow the mixing of the components 3. In particular, the mixing phases can be conducted at a blade speed between 15 and 25 revolutions per minute. The speed of the counter-blades in these phases can range between 50 and 60 revolutions per minute.

The process preferably includes a second mixing phase, following the first mixing phase, wherein a second mixture **101** is prepared. It is obtained by adding Disteardimonium Hectorite 315 to the first mixture 100 and subsequently mixing. The addition can be carried out inside the same turboemulsifier. This mixing phase can be conducted at a blade rotation speed higher than that of the first mixing phase. As in the previous phase, the second mixing phase also takes place at a temperature preferably between 75°C and 95°C, more preferably between 80°C and 90°C.

The second mixture 101 preferably contains Disteardimonium Hectorite 315 homogeneously dispersed, without undispersed clumps.

At this point, the process includes a third mixing phase wherein a third mixture **102** is prepared. It is obtained by adding Microcrystalline Wax 304, Synthetic Wax 305 to the second mixture 101, and subsequently mixing them. This phase can be conducted under the same blade rotation speed and temperature conditions as the second mixing phase.

The third mixing phase ends when complete dissolution of the waxes in the third mixture 102 is achieved.

The third mixing phase can be followed by a cooling phase wherein the third mixture 102 is brought to a temperature preferably between 35°C and 45°C, more preferably between 37°C and 43°C. During the cooling phase, the blade rotation speed can be reduced to a speed lower than that of the first mixing phase.

At this point, the process includes a fourth mixing phase wherein a fourth mixture **103** is prepared. The fourth mixture is obtained by adding Tocopheryl Acetate 316 and Synthetic Fluorphlogopite 318 to the third mixture 102, followed by mixing them. This phase preferably takes place at a temperature between 35°C and 45°C, more preferably between 37°C and 43°C. Therefore, the temperature can be maintained constant after the cooling phase. The blade rotation speed is preferably comparable to that of the first mixing phase.

The process advantageously includes a phase of adding Stevioside 2. It imparts at least a sweetening effect to the cosmetic balm 1.

As previously mentioned, to prevent the "scrub" effect due to the use of the formulation 1a, the Stevioside 2 is finely dispersed within it. To this end, the Stevioside 2 undergoes at least one size-reduction stage before the addition phase. Specifically, the size-reduction stage preferably includes micronisation. Micronisation can be carried out using a cyclone filter. Therefore, Stevioside 2 is introduced as raw granular material and micronized inside the cyclone, which causes the Stevioside 2 particles to break apart through mutual collisions once they are introduced into a gas flow. The powders are directed against the walls of the cyclone filter and sorted by size due to gravity, which causes them to settle at different heights based on their weight. Air jets from above select size ranges of the powders.

Furthermore, the process preferably includes a sieving phase for Stevioside 2 before the addition phase. Specifically, the sieving phase can be carried out using a sieve mesh with a mesh size preferably between 0.1 mm and 0.3 mm. More preferably, they are between 0.15 mm and 0.25 mm. Even more preferably, sieving can be performed with a mesh of at least 200 mesh. This way, a finer size is selected, making it easier to incorporate into anhydrous formulations without causing a scrub effect, but only a sweetening effect. Specifically, Stevioside 2, before the addition phase, comprises grains preferably smaller than 70 µm. More preferably, the grain size is smaller than 65 µm.

The formulation 1 preferably contains a percentage of Stevioside 2 less than 1%. This amount is advantageous for achieving the desired effect.

In the addition phase, the formulation 1 is preferably obtained by adding to the fourth mixture 103 Tribehenin 311, Ethylhexyl Palmitate 312, Sorbitan Isostearate 313, Lactic Acid 314, Palmitoyl Tripeptide-1 5, Theobroma Grandiflorum Seed Butter 320, Tocopherol 310, Orbignya Oleifera Seed Oil 317, Tetrahexyldecyl Ascorbate 319, and Stevioside 2. The addition is then followed by mixing.

This phase preferably takes place at a temperature between 35°C and 45°C, more preferably between 37°C and 43°C. The blade rotation speed is maintained the same as in the previous phase.

In the addition phase, additives **4** are preferably added. These have at least one of the following effects: colouring, fragrance, flavouring.

In particular, the additives 4 include at least one of the following: Blackberry Aroma **400,** Geraniol **401,** Melon Fragrance **402,** Salted Caramel Fragrance **403,** Sweet Vanilla Fragrance **404,** Caramel Candy Fragrance **405,** CI 77499 **406,** Cl 77491 **407,** CI 15850 **408,** CI 42090 **409,** CI 19140 **410,** CI 77891 **411,** CI 45410 **412.** The additives 4 such as Blackberry Aroma 400, Geraniol 401, Melon Fragrance 402, Salted Caramel Fragrance 403, Sweet Vanilla Fragrance 404, Caramel Candy Fragrance 405 impart fragrance or flavouring effects to the formulation 1. Combinations of additives 4, having fragrance or flavouring effects, in different proportions, can be combined to create new fragrances.

The additives 4 such as CI 77499 406, Cl 77491 407 (both iron oxides), CI 15850 408, CI 42090 409, CI 19140 410, Cl 77891 411 (titanium dioxide), CI 45410 412 have a colouring effect. Specifically, CI 19140 410 imparts a yellow colouring to the formulation 1, CI 15850 408 a red colouring, CI 77891 411 a white colouring, CI 42090 409 a blue colouring, CI 77499 406 a brownish colouring, CI 77491 407 a red colouring, and CI 45410 412 a fuchsia colouring. Various combinations of additives 4, with colouring effects, in different quantities, can produce different colouring. The additives 4 are preferably added in quantities corresponding to a percentage by weight of the formulation 1a of less than 2%.

Once the formulation 1a has been prepared, a phase of controlling the properties of the formulation can be performed. This control can be carried out both in terms of physical properties, as well as in comparison of the colouring and fragrance or aroma imparted to the formulation 1 through the addition of additives 4.

The cosmetic balm 1 and the process for making it according to the invention provide significant advantages.

In fact, the cosmetic balm 1 has the advantage of imparting to a product, such as a fluid lip balm, the sole sweetening effect due to the addition of Stevioside 2. Moreover, the cosmetic balm 1 is capable of separating the sweetening effect from the undesired scrub effect.

This characteristic of the cosmetic balm 1 is achieved through the new process, according to the invention, for making the cosmetic balm 1. Indeed, this process has the advantage of introducing Stevioside 2 in a manner that allows it to be finely dispersed within the formulation 1a, ensuring that the cosmetic balm 1 does not cause the scrub effect.

Another advantage of the process is the simplicity of the equipment required, as it only requires the equipment typically used for preparing powder-based cosmetic formulations.

The invention is susceptible to variations within the scope of the inventive concept defined by the claims.

Within this scope, all elements can be replaced by equivalent elements, and the materials, shapes, and dimensions can be varied as needed.

## Claims

1. A fluid cosmetic balm (1) for lips composed of an anhydrous cosmetic formulation (1a), **characterized in that** it comprises at least Stevioside (2) capable of imparting at least a sweetening effect to said formulation (1a), said Stevioside (2) being finely dispersed in said formulation (1a).

2. The balm according to the preceding claim, wherein said formulation (1a) includes Palmitoyl Tripeptide-1 (5), capable of imparting at least a volumizing, moisturizing, and wrinkle-reducing effect to said formulation (1a).

3. The balm according to any one of the preceding claims, wherein said formulation (1a) includes a plurality of components (3) capable of imparting active, emollient, skin-conditioning, viscosity-regulating, surfactant, thickening, fragrance, makeup, emulsifying, rheological modifier, antioxidant, texturizing, plumping, moisturizing, and soothing effects to said formulation (1a).

4. The balm according to the preceding claim, wherein said components (3) include:
- Hydrogenated Polyisobutene (300),
- Diisostearyl Malate (301),
- Butyrospermum Parkii Butter (302),
- Polybutene (303),
- Microcrystalline Wax (304),
- Synthetic Wax (305),
- Octyldodecanol (306),
- Hydrogenated Poly(C6-14 Olefins) (307),
- Polyglyceryl-2 Triisostearate (308),
- Phytosteryl/lsostearyl/Cetyl/Stearyl/Behenyl Dimer Dilinoleate (309),
- Tocopherol (310),
- Tribehenin (311),
- Ethylhexyl Palmitate (312),
- Sorbitan Isostearate (313),
- Lactic Acid (314),
- Disteardimonium Hectorite (315),
- Tocopheryl Acetate (316),
- Orbignya Oleifera Seed Oil (317),
- Synthetic Fluorphlogopite (318),
- Tetrahexyldecyl Ascorbate (319),
- Theobroma Grandiflorum Seed Butter (320).

5. A process for making a fluid cosmetic balm (1) for lips composed of an anhydrous cosmetic formulation (1a), comprising at least one phase of mixing a plurality of components (3)
and further **characterized in that** it comprises:
- a phase of adding Stevioside (2) capable of imparting at least a sweetening effect to said formulation (1a);
- said Stevioside (2) being subjected to at least one size-reduction stage before said addition phase.

6. The process according to the preceding claim, wherein said size-reduction stage comprises micronisation.

7. The process according to any one of claims 5 and 6, comprising a phase of sieving said Stevioside (2) before said addition phase.

8. The process according to any one of claims 5-7, wherein said formulation comprises at least Palmitoyl Tripeptide-1 (5).

9. The process according to any one of claims 5-8, wherein said components (3) include: Hydrogenated Polyisobutene (300), Diisostearyl Malate (301), Butyrospermum Parkii Butter (302), Polybutene (303), Microcrystalline Wax (304), Synthetic Wax (305), Octyldodecanol (306), Hydrogenated Poly(C6-14 Olefins) (307), Polyglyceryl-2 Triisostearate (308), Phytosteryl/lsostearyl/Cetyl/Stearyl/Behenyl Dimer Dilinoleate (309), Tocopherol (310), Tribehenin (311), Ethylhexyl Palmitate (312), Sorbitan Isostearate (313), Lactic Acid (314), Disteardimonium Hectorite (315), Tocopheryl Acetate (316), Orbignya Oleifera Seed Oil (317), Synthetic Fluorphlogopite (318), Tetrahexyldecyl Ascorbate (319), Theobroma Grandiflorum Seed Butter (320).

10. The process according to the preceding claim, comprising:
- a first mixing phase wherein a first mixture (100) is prepared by mixing said Diisostearyl Malate (301), said Hydrogenated Polyisobutene (300), said Polybutene (303), said Butyrospermum Parkii Butter (302), said Octyldodecanol (306), said Hydrogenated Poly(C6-14 Olefins) (307), said Polyglyceryl-2 Triisostearate (308), said Phytosteryl/lsostearyl/Cetyl/Stearyl/Behenyl Dimer Dilinoleate (309), and said Tocopherol (310);
- a second mixing phase following the first mixing phase, wherein a second mixture (101) is prepared by adding said Disteardimonium Hectorite (315) to said first mixture (100) and mixing said first mixture (100) and said Disteardimonium Hectorite (315);
- a third mixing phase wherein a third mixture (102) is prepared by adding said Microcrystalline Wax (304) and said Synthetic Wax (305) to said second mixture (101) and mixing said second mixture (101), said Microcrystalline Wax (304), and said Synthetic Wax (305);
- a fourth mixing phase wherein a fourth mixture (103) is prepared by adding said Tocopheryl Acetate (316) and said Synthetic Fluorphlogopite (318) to said third mixture (102) and mixing said third mixture (102), said Tocopheryl Acetate (316), and said Synthetic Fluorphlogopite (318);
and wherein, in said addition phase, said formulation (1) is obtained by adding to said fourth mixture (103) said Tribehenin (311), said Ethylhexyl Palmitate (312), said Sorbitan Isostearate (313), said Lactic Acid (314), said Palmitoyl Tripeptide-1 (5), said Theobroma Grandiflorum Seed Butter (320), said Tocopherol (310), said Orbignya Oleifera Seed Oil (317), and said Tetrahexyldecyl Ascorbate (319), as well as said Stevioside (2), followed by mixing.

11. The process according to any one of claims 5-10, wherein in said addition phase, additives (4) having at least one of the effects of colouring, fragrance, or flavouring are added, said additives (4) comprising at least one of the following:
Blackberry Aroma (400), Geraniol (401), Melon Fragrance (402), Salted Caramel Fragrance (403), Sweet Vanilla Fragrance (404), Caramel Candy Fragrance (405), CI 77499 (406), Cl 77491 (407), CI 15850 (408), Cl 42090 (409), CI 19140 (410), CI 77891 (411), CI 45410 (412).

12. The process according to claims 8 and 9 and any one of claims 5-7, 10, and 11, wherein the formulation (1a) contains a percentage by weight of:
- said Stevioside (2) less than 1%,
- said Palmitoyl Tripeptide-1 (5) less than 1%,
- said Hydrogenated Polyisobutene (300) between 30% and 40%,
- said Diisostearyl Malate (301) between 20% and 30%,
- said Butyrospermum Parkii Butter (302) between 9% and 15%,
- said Polybutene (303) between 5% and 15%,
- said Microcrystalline Wax (304) between 2% and 7%,
- said Synthetic Wax (305) between 1% and 5%,
- said Octyldodecanol (306) between 1 % and 4%,
- said Hydrogenated Poly(C6-14 Olefins) (307) between 1% and 4%,
- said Polyglyceryl-2 Triisostearate (308) between 1% and 4%,
- said Phytosteryl/lsostearyl/Cetyl/Stearyl/Behenyl Dimer Dilinoleate (309) less than 2%,
- said Tocopherol (310) less than 1%,
- said Tribehenin (311) less than 1%,
- said Ethylhexyl Palmitate (312) less than 2%,
- said Sorbitan Isostearate (313) less than 1%,
- said Lactic Acid (314) less than 1%,
- said Disteardimonium Hectorite (315) less than 2%,
- said Tocopheryl Acetate (316) less than 1%,
- said Orbignya Oleifera Seed Oil (317) less than 1%,
- said Synthetic Fluorphlogopite (318) less than 1%,
- said Tetrahexyldecyl Ascorbate (319) less than 1%, and
- said Theobroma Grandiflorum Seed Butter (320) less than 1%.

13. The process according to claim 8 and any one of claims 4-6, 8, and 9, wherein each phase is conducted at a pressure between 700 mbar and 1100 mbar, and said first, said second, and said third mixing phases take place at a temperature between 75°C and 95°C, while said fourth mixing phase and said addition phase take place at a temperature between 35°C and 45°C.

14. The process according to at least claim 5, wherein said Stevioside (2), before said addition phase, consists of grains having a size smaller than 70 µm.
